# EUROPEAN PATENT SPECIFICATION

(11) **EP 1 605 838 B1**
(45) Date of publication and mention of the grant of the patent: **17.06.2009**
(21) Application number: 04723118.8
(22) Date of filing: 24.03.2004
(51) Int. Cl.: A61B 17/17, A61F 2/46

(54) **SYSTEM FOR ENSURING CORRECT INSERTION OF AN ARTIFICIAL HIP JOINT**
SYSTEM ZUR POSITIONSGENAUEN EINFÜHRUNG EINES KÜNSTLICHEN HÜFTGELENKS
SYSTEME PERMETTANT D'ASSURER L'INSERTION CORRECTE D'UNE ARTICULATION COXOFEMORALE ARTIFICIELLE

(30) Priority: 24.03.2003 NO 20031333
(43) Date of publication of application: 21.12.2005
(73) Proprietor: OM Surgical (UK) Limited, London SW62JR (GB)
(72) Inventor: IVERSEN, Björn Franc, 3050 Humlebaek (DK)
(74) Representative: Chapman, Paul William
(86) International application number: PCT/NO2004/000085
(87) International publication number: WO 2004/084740

(56) References cited:
- WO-A-01/30247
- WO-A-02/080824
- US-B1- 6 395 005

## Description

The present invention relates to the area of orthopaedic surgery, and in particular to a system for ensuring that prosthesis components or parts thereof are inserted correctly upon implantation of artificial hip joints and to methods for ensuring correct insertion of the parts of an artificial hip joint or femoral prosthesis during surgery.

An artificial hip joint has two main components; a prosthesis stem and a socket which is often referred to as "the cup". One end of the prosthesis stem is provided either with a spherical ball head or a prosthesis neck, which may be modular and designed so that the neck may rotate in the stem, on which stem a ball head may be placed. Said ball head is designed for a close, sliding fit in a spherical recess in the cup. Together, the prosthesis stem/neck with the ball head and the cup will act as a ball joint to replace the natural hip joint.

The other end of the prosthesis stem comprises an elongated part designed to be mounted in the hollow internal canal in the patient's femur.

The cup is designed to be attached in the natural joint socket on the patient's pelvis. The hemispherically shaped, recess in the cup is linked with an external (outer) surface designed to be attached to the pelvis, via a side face. The external surface may have various shapes, all according to the method of attachment to the pelvis and other choices made by the supplier. Several of the cups that are in use are shaped as an approximate hemisphere, where the outer hemispherical surface is designed to be cemented to the pelvis. The side face that connects the recess and the exterior surface may be flat or possibly inwardly sloping towards the recess, which is preferably approximately centered in the side face.

The prosthesis stem and the cup may be fixed to the femur and the pelvis respectively by using cement, or through a cement-free force fit. The invention is intended for and may be used with both fixation techniques but is only described in detail for the cemented version.

When replacing a worn out hip with a prosthesis, the cavity on the pelvis is milled out to receive the cup, which is then fixed either by means of poly methyl metacrylate cement or force fit.

The head of the femur is replaced. This is done by dividing the neck of the femur and evacuating approximately the cranial 1/3 of the femoral canal to make room for the elongated prosthesis stem that is either cemented or force fitted into the internal canal.

If the ball head is detachable, this is placed on the prosthesis neck before the ball head is placed in the cup, the joint is assembled by lifting the patient's leg up to a natural position and inserting the ball head in the recess in the cup, whereupon the incision is closed.

Ideally such prostheses should provide the patients with mobility that approximates that which is provided by the natural joint. However, as the tension of the soft tissues surrounding the joint, e. g. the joint capsule etc. is weakened after the operation is possible for the patient to place the leg in a position outside its normal range of movement. This may cause the head of the prosthesis to lever out of the cup (dislocation). Moreover, it is important that "natural" movements of the leg do not bring the joint in positions where the neck of the prosthesis rides on the edge of the cup as this may result in dislocation through simple leverage. Dislocation occurs in 1 to 9 % of all patients who have undergone total hip replacement. If this happens, the patient must be anaesthetised before the joint may be reduced. In some patients a reoperation is required. The risk of dislocation is considered increased in patients whose prosthesis components are inserted in an incorrect mutual positioning, than in those where the mutual positioning of the components (i.e. the spatial interface between the components) is correct.

The inventor has previously shown that an optimal mutual relationship between the prosthesis neck and the cup under experimental conditions (not published) results in a reduced risk of dislocation because the patient can go through the everyday natural range of motion (ROM) without the parts of the prosthesis ending up in such mutual positioning so as to entail a risk of dislocation.

The inventor has previously shown (not published) that the most adequate ROM is achieved by assembling both prosthesis components in a manner so as to give them a forward angle of about 15 degrees relative to the frontal plane of the body, while the cup forms an angle of 45 degrees with the horizontal plane. In medical terminology, forward angling is termed anteversion, whereas a backward angling is termed retroversion. A cup angle greater than 45 degrees relative to the horizontal plane is termed abduction, whereas an angle less than 45 degrees is called adduction.

The inventor has also previously shown (not published) that even though the optimum is to have each of the components angled 15 degrees forward, the resulting Range of Motion of the prosthetic joint is nearly as good if the sum of the forward angling of the two components is 30 degrees. Thus a prosthesis joint where the cup is angled forwards at 10 degrees and the prosthesis stem is angled forwards at 20 degrees will result in a ROM for the patient that is nearly as adequate as if both components were angled forward at 15 degrees, the sum of the forward angling being 30 degrees is both cases. During the fixation of the prosthesis stem, accurate alignment of the prosthesis stem in the femoral canal may be difficult in practice, especially if the stem is to be fastened cement free. Due to the shape of the internal canal in femur, the prosthesis stem has a tendency to orient itself in accordance with the natural shape of the canal in femur resisting to be forced into the specific angle intended by the surgeon..

Several solutions for insertion of the cup respectively the prosthesis stem and to ensure that the individual part is being fixed correctly are known.

WO 01/30247 discloses an apparatus and method for quantifying the spatial relationship between a landmark on the ilium and a landmark on the femur.

A device for alignment and for holding the cup as it is cemented into the pelvis is known from US 5.976. 149. The temporarily holding device for the cup is temporarily fixed to the pelvis during the cementation.

From GB 2.197.790 a device for assuring that the cup in an artificial hip joint is fixed with a predetermined anteversion and a predetermined angle to the horizontal plane, is known. The mutual angle between the parts in the prosthesis is not taken care of by using this device.

Instruments for insertion of the cup are described in EP 888.759 Al and US 5.540. 697. These instruments are handles onto which the cup is fastened during the insertion but they do not have any means for assuring the correct position and direction of the cup. Thus, these parameters depend on the individual surgeon, his visual assessment and his experience.

Several devices and means for assuring alignment of the prosthesis stem during the insertion into the femur is know from EP 207 873, PCT/DE90/00715 and EP 865 776 A2. As mentioned this fixation is not critical. Additionally, these publications do only describe devices and means for insertion of one of the prosthesis parts, i.e. the prosthesis stem, and does not describe any means to ensure an intended mutual angle between the cup and the prosthesis stem.

The inventor's own WO 01/19296 relates to a tool to set the intended mutual angle between the prosthesis stem and the cup during the cementation of the cup in the pelvic cavity. The tool described may be locked relative to the prosthesis stem and has one or more abutment surface (s) designed to rest against a surface of the cup so that the parts are locked relative to each other. Preferably the prosthesis or its corresponding rasp is fixed to the channel in femur firstly, before the leg of the patient and the prosthesis stem and the device are placed in a normal position and is used to position the cup correctly. This device, however, may not be used by itself to assure the mutual positioning between the prosthesis parts when using cups to be mounted without the use of cement. Additionally it may only be used to assure that the parts of the prosthesis is positioned correctly relative to each other, but does not take into consideration the correct insertion relative to the patient.

The bone coverage for the cup is often inferior when the cup is correctly mounted. The surgeon will often in cases like that choose to deviate from the normally desired angle for the cup to get a better bone coverage. In these cases it would be of great advantage if the surgeon could measure the actual angle and thus be able to choose the best compromise between angle and bone coverage.

The present inventor's own WO02/080824 describes a computer based method and means that ensure a correct mutual positioning of the main parts of the prosthesis in order to reduce the possibility of errors, and thereby also reduce the risk of dislocation with the resulting pain for the patient, and a possible second operation.

The method and tool according to WO02/080824 also makes it possible to accurately measure required adjustments of the length of the limb by inserting the prosthesis so that the resulting leg length may be lengthened or shortened, and to adjust offset, i.e. the distance between the longitudinal axis of the femur and the sagittal plane of the body.

The desired adjustment of the offset and/or length of the limb will be determined during a pre-operative examination and outpatient examination of the patient and the patient's radiograms.

This assessment may be sufficient, especially for experienced surgeons who carry out a considerable number of this type of operation each year. But it is estimated that 80% of all implantations of artificial hip joints are carried out by surgeons who do less than 20 of these per year. This number is not sufficient to obtain and maintain the skills and routine required to achieve good surgical results. It is therefore desirable to have a method and means that ensure a correct mutual positioning of the main parts of the prosthesis in order to reduce the possibility of errors, and thereby also reduce the risk complications including dislocation with the resulting pain for the patient, and a possible second operation.

The computer based tool according to WO02/080824 is, however, relatively expensive and complicated and requires high technical skills besides the surgical skills. This type of tool is therefore normally utilized by the larger hospitals and mainly university hospitals.

Tools for ensuring the correct insertion of an artificial hip joint, so that the prosthesis is correctly positioned relative to the femur and pelvis and that the parts of the artificial joint are correctly placed relative to each other, are of special importance in minimally invasive surgery. In minimally invasive surgery the operation is performed through relatively small incisions. The main advantage with minimal invasive surgery is that the damage done to healthy tissue is reduced and that the convalescence period becomes shorter. Working through small incisions is, however, more demanding than traditional surgery. For hip joint prosthesis surgery the main problem is related to the position of the parts of the prosthesis. There is a necessity for a method and tools to ensure that the artificial hip prosthesis is correctly inserted during the surgery.

Recently a number of high cost electronic navigation systems for achieving correct alignment of hip prostheses has been developed. As these systems are expensive, complicated and demanding to use they are mainly employed in a limited number of university clinics, while smaller hospitals cannot afford this technology or have not the specially trained human resources to man them. Thus, an inexpensive mechanical invention like the present is in demand to solve the prevailing complications involved with misalignment of the prosthetic components and to spread minimally invasive surgery beyond university clinics.

A measuring device according to the present invention is defined in claims 1 to 9. According to a second aspect of the invention, a system is provided for ensuring correct insertion and spatial orientation of a prosthesis cup and/or a prosthesis stem of an artificial hip joint, the system comprising:
a) a tool for controlling the mutual positioning of the main components in a hip prosthesis;
b) a measuring device as defined in any of claims 1 to 9; and
c) a detachable positioning tool designed to be connected to a handle part connected to the tool according to item a) or to extensions of the prosthesis components, and to the two supports connected to the patients' pelvis and leg.

A measuring device may be provided for measuring the distance between two supports for use during surgical procedures, where said supports are connected to bones in the patient's body, wherein the measuring device comprises an elongated main body;
a first arm connected substantially perpendicular to the longitudinal axis of and close to one end of said main body;
an adjustable member displaceably connected to said main body;
a second arm being substantially parallel to said first arm, connected to the adjustable body; connection members arranged at one of the ends of said first and second arm, where said connection members are adapted to interact with receptors at the supports.

Said first arm may be displaceably connected to the main body in a direction substantially perpendicular to the longitudinal axis of said main body.

Said second arm may be displaceably connected to the adjustable member in a direction substantially perpendicular to the longitudinal axis of said main body.

When using the measuring device, the connection members are brought in contact with the receptors at both supports. The adjustable member and the length of said first or said second arm is then adjusted so that the connection members and the corresponding receptor rests are in full contact with each other. In the illustrated embodiment where the receptors are grooves, the position of the adjustable body and the adjustable arm are adjusted until the connection members rest in the grooves and is in contact with the bottom of the groove at most or all of the length of the connection members It is preferred that the adjustable member comprises means to lock the adjustable member in a preferred position along the main body. By locking the adjustable member relative to the main body, unintentional movement of the adjustable body relative to the main body after performing the measurement is avoided.

Preferably, the adjustable member is adapted to receive and interact with a locking member to lock the adjustable member to the main body.

Preferably, the measuring device comprises means to lock said first arm or said second arm in preferred positions. This is done to avoid unintentional movement of the adjustable arm, i.e. the first arm or the second arm, after performing the measurements.

According to a preferred embodiment, substantially parallel bores are made in the main body and the adjustable body. Said bores provides firm fixation for drill sheaths in using the measuring device as a drill guide and for correct positioning of screws into the patients' femur and pelvis and for fixation of the supports including the receptors.

A positioning tool may be provided for ensuring correct insertion of a prosthesis cup and/or a prosthesis stem of an artificial hip joint, the tool comprising:
a handle part adapted to be releasably connected to a tool for controlling the mutual position of the main parts of a hip prosthesis; and
two or more flexible arms, where each arm close to a first end thereof, is adapted to be releasably connected to said handle part and close to a second end thereof, is connected to a connection member that is adapted to interact with receptors at the supports, and where the flexible arms comprise means to lock the arms in a wanted configuration.

The flexible arms may comprise arm members (8,9) that are rotably connected to each other about an axis that are substantially perpendicular to the arm members.

The flexible arms may comprise arm members that are telescopically connected to each other.

It is preferred that the positioning tool comprise means to lock the relative position of the arm members in a desired position. Locking of the relative position of the arm members makes it possible to use the arms to re-establish the position of the prosthesis parts relative to the supports moving the prosthesis part in question until the connection member at the end of the arm rests in the groove of the support in question.

To ensure the correct positioning of the main parts of an artificial hip joint, i.e. a prosthesis stem and a cup, a method comprising the following steps may be provided:
a) placing the patient in a well defined start position;
b) preparing for fixation of supports to the patient's femur and pelvis, respectively;
c) fixation of said supports to the patients leg and pelvis;
d) measuring the mutual spatial position of said supports by means of a measuring device while the patient lies in said well defined start position;
e) making the surgical incisions, dividing the femoral neck, preparing the femur and pelvis for receiving the prosthesis parts;
f) inserting temporarily said main parts of, or trial components of, the prosthesis together with a tool for ensuring correct mutual position of the main parts of the prosthesis components;
g) again placing the patient in said well defined start position and controlling the start position of the patient, corrected for any planned change in leg length or offset, by means of said measuring device;
h) if possible, ensuring that the main parts of the prosthesis, or the trial components, are in correct position relative to each other and to the leg and pelvis, respectively, by inspecting the position;
i) connecting a position device according to claim 11 to the tool for ensuring correct mutual position of the main parts of the prosthesis or; connecting a position device according to claim 11 to prosthesis or rasp or an extension thereto;
j) placing the connection members of the flexible arms in receptors on said supports;
k) locking the flexible arms in a position where the connection members are placed in said receptors;
l) removing the tools and prosthesis parts;
m) inserting the prosthesis cup into the pelvis for force fit or together with cement to fasten the stem using the tool for ensuring mutual position of the parts of the prosthesis and using the flexible arm used in step i) -k) above to connect to the support at the pelvis to control that the prosthesis stem has the same position relative to said support as during step k);
n) in cemented techniques, waiting until the cement is sufficiently cured to remove the tools;
o) inserting the prosthesis stem into the femur for force fit or together with cement to fasten the stem using the tool for ensuring mutual position of the parts of the prosthesis and using the flexible arm used in step i) -k) above to connect to the support at the femur to control that the prosthesis stem has the same position relative to said support as during step k);
p) in cemented technique, waiting until the cement is sufficiently hardened to remove the tools; q) removing the tool for ensuring the correct mutual position and reassembling the prosthesis and finalizing the surgical procedure the conventional way.

It is preferred that said measuring device is used as a drilling guide during steps b) and c).

Preferably the surgery is performed by minimally invasive surgery. Minimally invasive surgery, allows the surgery to be performed making as small incisions as possible and thus damaging less soft tissue than traditional surgery.

The small incisions, however, makes it harder to navigate and place the femur, pelvis and prosthesis parts correctly relative to each other. The present tool allows control and reproducibility in the positioning of the parts of the prosthesis even during minimally invasive surgery.

During some surgical procedures an adjustment of the position of the adjustable body and/or the adjustable arm is performed after step d) before fixing the position of the adjustable arm and adjustable body, respectively, to adjust for planned changes in postoperative leg length and/or offset. Preferably, the measuring device is locked in its resulting position after measurement and any adjustments for leg length and/or offset. By locking the device after adjusting for leg length and/or offset, unintentional movement of the parts of the measuring device is avoided.

A method may be provided for ensuring correct positioning of the main parts of an artificial hip joint, i.e. an prosthesis stem and a cup, wherein the method comprises the following steps:
r) placing the patient in a well defined start position;
s) preparing for fixation of supports to the patient's femur and pelvis, respectively;
t) fixation of said supports to the patients leg and pelvis;
u) measuring the mutual position of said supports by means of a measuring device while the patient lies in said well defined start position;
v) making the necessary incisions, dividing the femoral neck, preparing the femur and pelvis for receiving the prosthesis parts;
w) inserting the prosthesis stem into the femur and the cup both without or together with cement to fasten them into the femur and pelvis using the tool for ensuring mutual position of the parts of the prosthesis to ensure correct mutual positioning;
x) replacing the patient in said well defined start position and controlling the position by means of said measuring device;
y) in procedures where cement is used for fixation, waiting until the cement is sufficiently hardened to remove the tools;
z) removing the tool for ensuring the correct mutual position and reassembling the prosthesis and finalizing the surgical procedure the conventional way.

To ensure the correct positioning and spatial orientation of the main parts of an artificial hip joint, i.e. an prosthesis stem, neck and cup, a method comprises the following steps may be provided:
aa) placing the patient in a well defined start position;
bb) preparing for fixation of supports to the patient's femur and pelvis, respectively;
cc) fixation of said supports to the patients leg and pelvis;
dd) making the surgical incisions, dividing the femoral neck, preparing the femur and pelvis for receiving the prosthesis parts;
ee) inserting temporarily said main parts of the prosthesis together with a tools for ensuring correct mutual position of the main parts of the prosthesis;
ff) if possible ensuring that the main parts of the prosthesis are in correct position relative to each other and to the femur and pelvis, respectively, by inspecting the position;
gg) connecting a position device according to claim 11 to the tool for ensuring correct mutual position of the main parts of the prosthesis;
hh) placing the connection members of the flexible arms in receptors at said supports;
ii) locking the flexible arms in a position where the connection members are placed in said receptors;
jj) removing the tools and prosthesis parts;
kk) inserting the prosthesis stem into the femur together with or without cement to fasten the stem using the tool for ensuring mutual position of the parts of the prosthesis and using the flexible arm used in step gg) to ii) above to connect to the support at the femur to control that the prosthesis stem has the same position relative to said support as during step ii);
11) in cemented procedures, waiting until the cement is sufficiently cured to remove the tools;
mm) inserting the prosthesis cup into the pelvis without or together with cement to fasten the stem using the tool for ensuring mutual position of the parts of the prosthesis and using the flexible arm used in step gg) and ii) above to connect to the support at the pelvis to control that the prosthesis stem has the same position relative to said support as during step ii);
nn) if applicable, waiting until the cement is sufficiently cured to remove the tools; oo) removing the tool for ensuring the correct mutual position and reassembling the prosthesis and finalizing the surgical procedure the conventional way.

To ensure the correct positioning of the main parts of an artificial hip joint, i.e. a prosthesis stem and a cup, where the stem is a modular stem where the neck of the stem may be adjusted relative to the rest of the stem, a method comprising the following steps may be provided:
pp) placing the patient in a well defined start position;
qq) preparing for fixation of supports to the patient's femur and pelvis, respectively;
rr) fixation of said supports to the patients leg and pelvis;
ss) measuring the mutual spatial position of said supports by means of a measuring device while the patient lies in said well defined start position;
tt) making the surgical incisions, dividing the femoral neck, preparing the femur and pelvis for receiving the prosthesis parts;
uu) inserting temporarily said main parts of, or trial components of, the prosthesis together with a tool for ensuring correct mutual position of the main parts of the prosthesis components;
vv) again placing the patient in said well defined start position and controlling the start position of the patient, corrected for any planned change in leg length or offset, by means of said measuring device;
ww) if possible, ensuring that the main parts of the prosthesis, or the trial components, are in correct position relative to each other and to the leg and pelvis, respectively, by inspecting the position;
xx) connecting a position device according to claim 11 to the tool for ensuring correct mutual position of the main parts of the prosthesis or; connecting a position device according to claim 11 to prosthesis or rasp or an extension thereto;
yy) placing the connection members of the flexible arms in receptors on said supports;
zz) locking the flexible arms in a position where the connection members are placed in said receptors;
aaa) removing the tools and prosthesis parts;
bbb) inserting the prosthesis cup into the pelvis for force fit or together with cement to fasten the stem using the tool for ensuring mutual position of the parts of the prosthesis and using the flexible arm used in step xx) -zz) above to connect to the support at the pelvis to control that the prosthesis stem has the same position relative to said support as during step xx);
ccc) in cemented techniques, waiting until the cement is sufficiently cured to remove the tools;
ddd) inserting the prosthesis stem into the femur for force fit or together with cement to fasten the stem and if applicable allow the cement to harden;
eee) using the tool for ensuring mutual position of the parts of the prosthesis and using the flexible arm used in step xx)-xx) above to connect to the support at the femur and adjusting the modular neck so that the prosthesis stem has the same position relative to said support as during step mm);
fff) removing the tool for ensuring the correct mutual position and reassembling the prosthesis and finalizing the surgical procedure the conventional way.

However, these methods do not form part of the invention, and are merely provided by way of background information.

In the following, the invention will be described further with reference to the attached figures, in which:
**Figure 1** shows a preferred embodiment of the tool according to the present invention connected to an anteversion head;

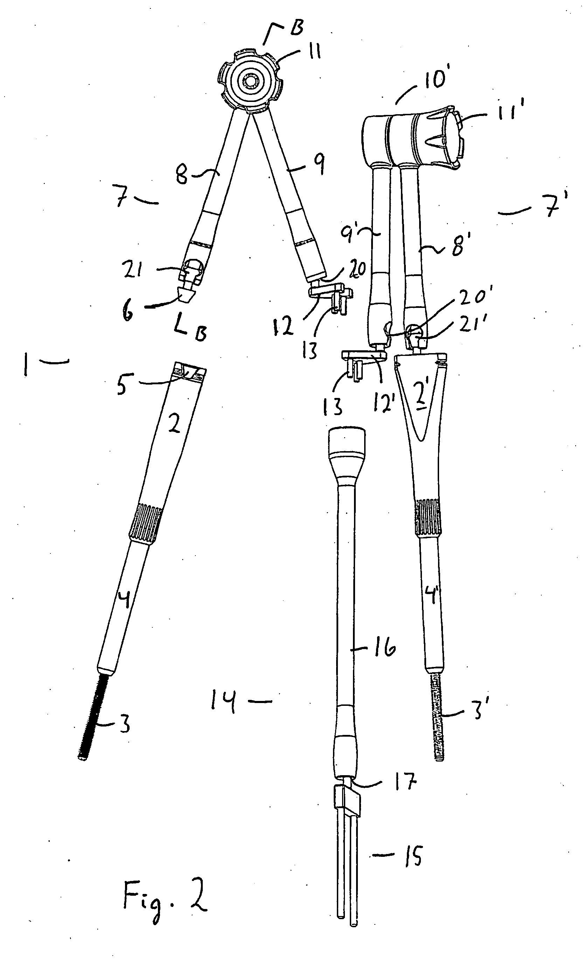

**Figure 2** shows the same tool as fig. 1, wherein main parts are partly disconnected;
**Figure 3** is a section view of a arm member of the tool seen along the line B-B;
**Figure 4** shows the same tool as fig. 1, partly disassembled;

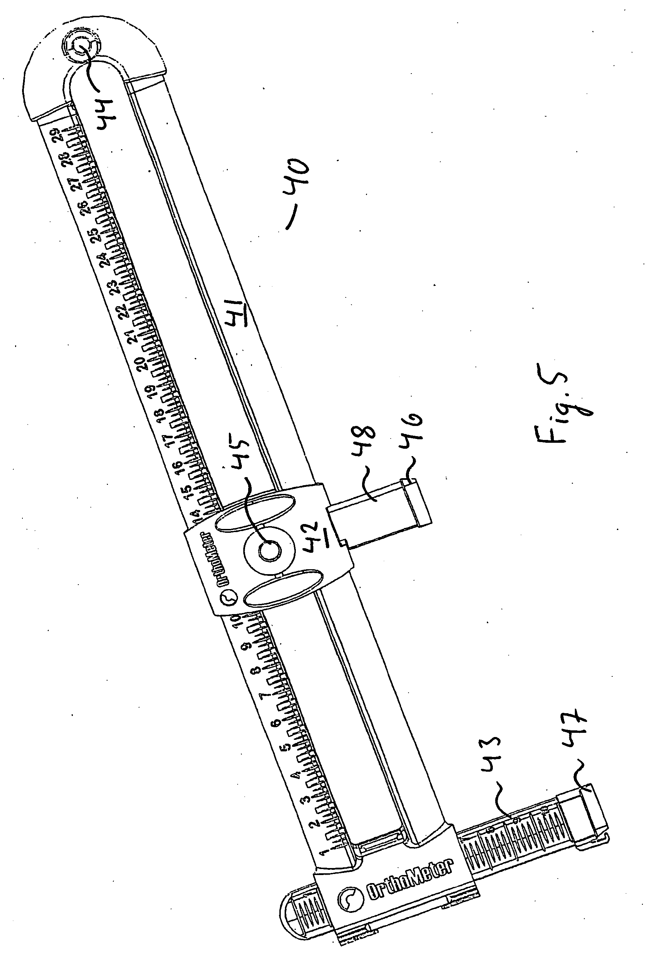

**Figure 5** shows an alternative embodiment;

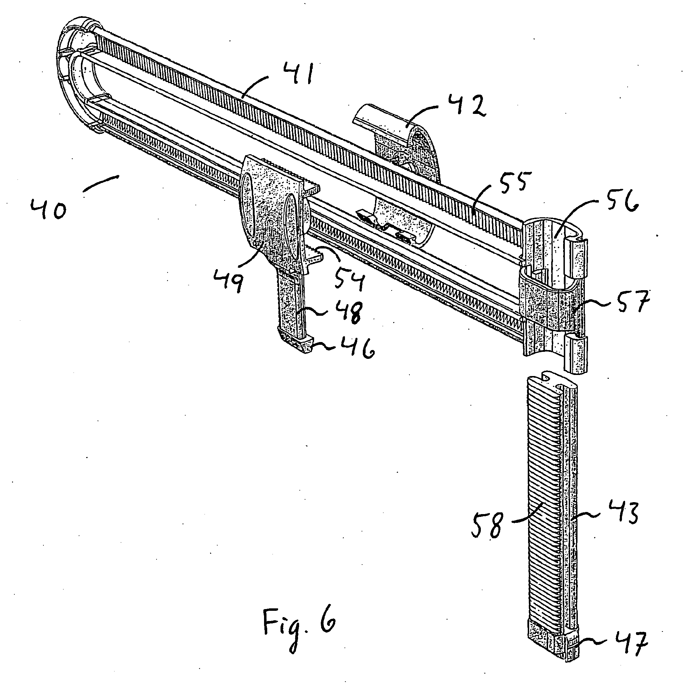

**Figure 6** shows an alternative tool according to the invention;

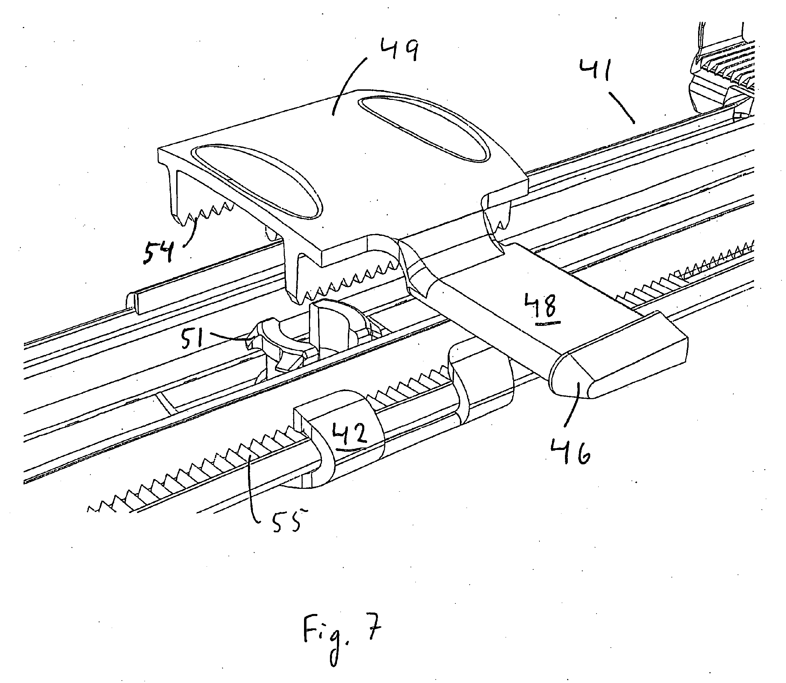

**Figure 7** shows the tool of fig. 7 set on the prosthesis stem; and

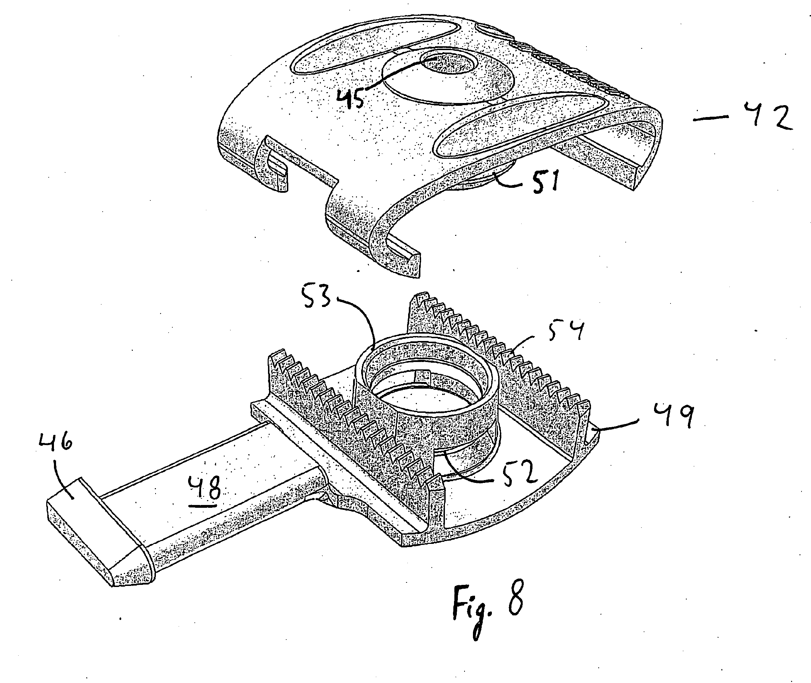

**Figure 8** shows an alternative embodiment of the present tool, where the tools parts are disassembled.

### Detailed description of the invention

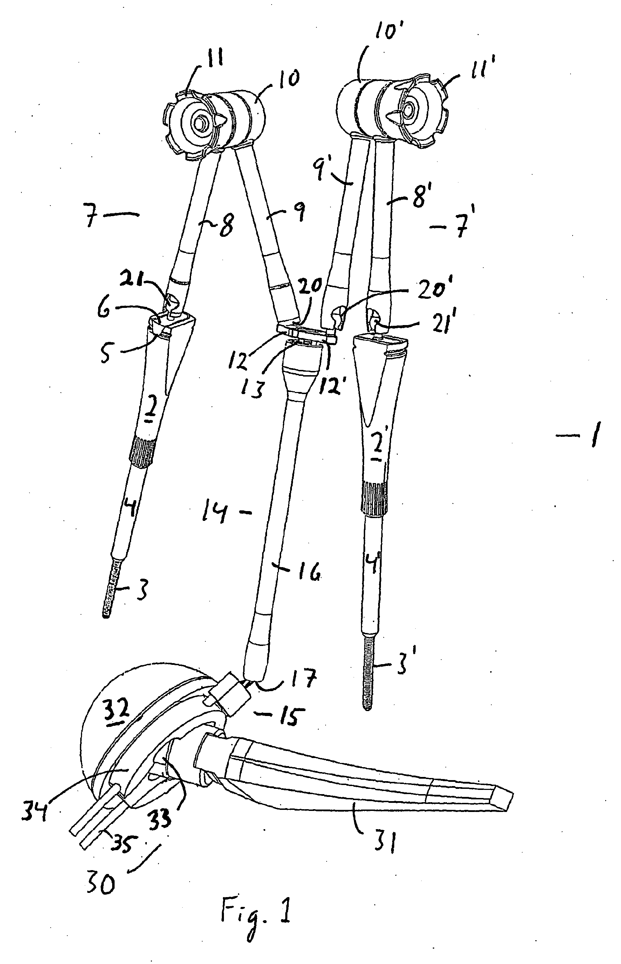

Figure 1 illustrates a positioning tool 1. The positioning tool 1 comprises two supports 2,2', a handle part 14 and two flexible arms 7,7'. The supports 2,2' are fastened in a conventional way to the femur and the pelvis, respectively, by means of screws 3, 3'. The screws may alternatively be substituted by pins. To avoid or reduce damage to soft tissue a protective sleeve 4,4' are placed around the part of the screws that are in contact with soft tissue. An alternative method for fastening the supports is by means of clamps that are clamped to a bone.

The handle part 14 comprises a stem 16 and a fork-member 15. The handle part 14 substitutes the handle in the tool 30 according to WO 01/19296 (anteversion head), wherein the fork portion is designed for interaction with the anteversion head 30 as described in the mentioned publication.

The fork member 15 is connected to the stem by means of a ball joint 17. The ball joint 17 may be locked in a wanted angle by means of a not shown push rod inside the stem 16 controlled by a not shown screw in the opposite end of the stem relative to the ball joint 17. When the push rod is pushed against the ball in the ball joint, the position of the fork member 15 relative to the stem becomes temporarily locked. In an alternative embodiment the fork member 15 and stem 16 are fixed relative to each other. A tool set may then include 2 or more combined fork and stem units having different angle between the parts so that the surgeon may choose the unit having the appropriate angle.

At the opposite end of the stem relative to the ball joint 17, the stem is provided with means to temporarily and removable connect the stem 16 to the flexible arms 7, 7'. The means to temporarily and removable connect the stem to the flexible arms are in the illustrated embodiment bores (not shown) at the end of the stem designed to interact with corresponding pins 13 at a connection plate 12,12'on the arms 7,7'. The pins 13 and the bores are designed so that the position of the arms relative to the stem are unambiguously defined when the pins are in the bores. The pins at the first arm 7 are also different from the pins at the second arm 7', either in relative position or in shape or dimension of the pins, so that it is impossible to mix up the arms.

Connection members 6,6' are provided at the opposite end of the flexible arms 7,7'. The connection members 6,6' are designed to fit into receptors 5,5'at the top of the supports 2,2'. The receptors 5,5' are formations that can interact with the connection members, such as grooves, ridges or other suitable formations. In the preferred embodiment, the receptors 5,5' are grooves at the top of the supports 2,2'. According to one alternative embodiment one of or both receptors may be provided with a graded scale in one or more directions. This may be obtained e. g. by using a ball shaped receptor having graded scales. This allows the surgeon to adjust the direction of the flexible arms in a controlled way, if necessary.

Each of the illustrated flexible arms 7,7' comprise a first 8,8'and a second 9,9'arm member. The arm members are rotary connected in a rotary link 10,10', which is rotary about an axis perpendicular to the longitudinal axis of arm members, close to one end of the arm members. At their free end, the arm members 8,8', 9,9' are connected to the connection plates 12,12' and the connection members 6,6' respectively, by means of ball joints 20,20', 21,21'.

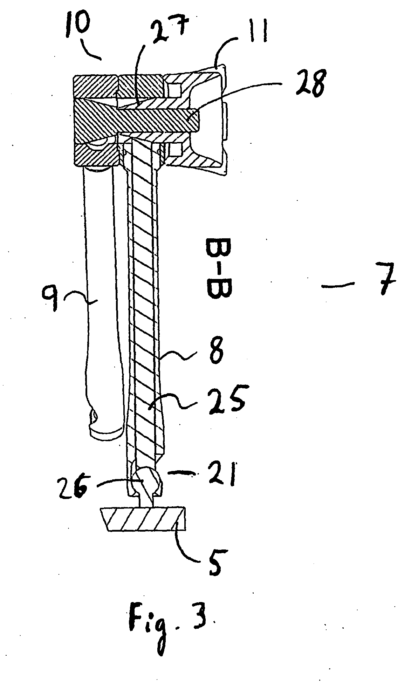

The rotary links 10,10' are adjustable by means of control wheels 11,11'. Figure 3 shows the section B-B of the first arm member 8 and the rotary link 10 in figure 1.

The rotary link comprises a bolt 28 having a longitudinal axis coinciding with the axis of rotation of the rotary link. The bolt 28 runs through both arm members close to one end and mainly perpendicular to their longitudinal axis.

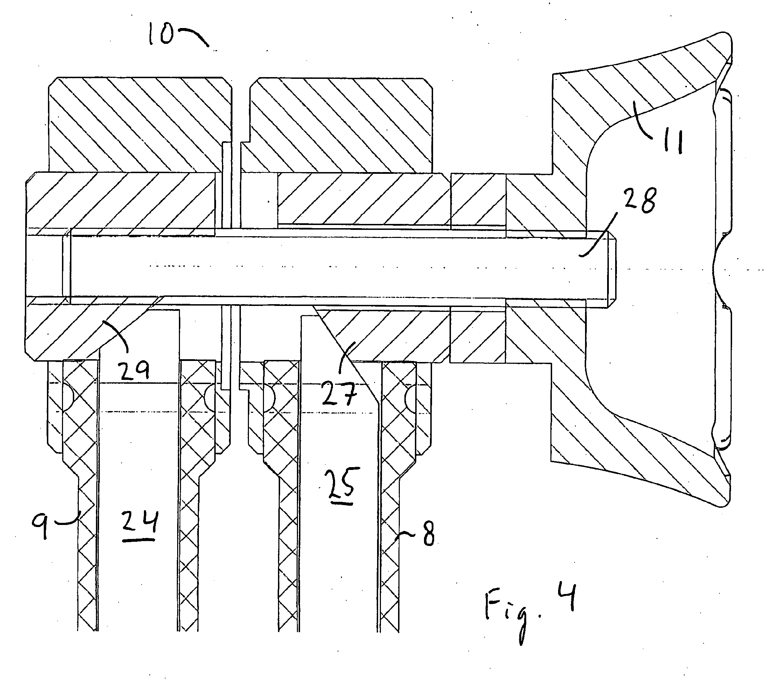

A push rod 25 is provided longitudinally inside the arm member 8 and rests against a ball 26 in the ball joint 21 at one end and against a conical body 27 in the rotary link 10. In the embodiment illustrated in figure 3 the conical body 27 is integrated in the control wheel 11, whereas the conical body 27 illustrated in figure 4, is a separate part. The control wheel 11 is screwed onto a bolt 28. The bolt 28 has a conical head 29. Alternatively may a conical body substitute the conical head 29.

When turning the control wheel the bolt one way the conical head and the conical body are pressed towards each other or removed from each other depending on the direction of rotation. When the control wheel is tightened, i. e. that the conical body and the conical head are forced towards each other, the conical body 27 is forced towards the push rod 25. The pushrod is then forced against the ball 26 of the ball joint 21 resulting in locking of the ball joint. At the same time the conical head of the bolt is forced towards a corresponding push rod 24 inside the second arm member 9 resting against a ball in the ball joint 20. Thus, the tightening of the control wheel 11 results in locking of the ball joints 20,21 and the rotational joint between the first arm member 8 and the second arm member 9 so that the arm 7 is fixed in a given configuration.

The joints 10,10', 20,20', 21,21'may be locked by other means than described above. The rotational links 10,10' may be locked as described using a wheel and a bolt to tighten the links. The ball joints 20,20', 21,21'might be locked by turning nuts that tightens and locks joint. The ball joints might also be locked by means of a lever operating an eccentric hinge exercising a force directly or indirectly to the balls of the ball joints to lock the ball in a wanted position.

The arms 7,7' may also be substituted by telescopically adjustable substantially straight arms. The mechanism for the telescopic adjustment is not vital as long as it is possible to lock the arm at a wanted length. The skilled man in the art is aware of different ways to lock a telescopically adjustable arm.

The tool 1 is preferably used in combination with a measuring device 40. The measuring device 40 comprises a lengthy main body 41, an adjustable member 42 and an adjustable arm 43. Two bores 44,45, one close to one end of the main body 41 and the other in the adjustable member, may serve as templates for making bores for fastening the screws 3,3'in femur and pelvis, respectively.

Connection members 46, 47 at an arm 48 at the adjustable member 42, and at the adjustable arm 43, respectively, are designed to rest against the receptors 5,5'at the supports 2,2' that are connected to the screws 3, 3'.

The adjustable member 42 may be moved along the main body 41. The adjustable member 42 may be locked to the main body 41 by means of a locking member 49 that are forced into engagement with adjustable member 42 and the main body 41. In the illustrated embodiment the arm 48 is a part of the locking member 49. The adjustable member 42 and locking member 49 are fastened to each other by means of hooks 51 mounted on the adjustable member 42 that are forced into engagement with notches 52 in a receiving member 53 on the locking member 49. The hooks 51 are not available for the user so that it is not possible to remove the locking member after the device is locked without breaking the locking member or the adjustable member. When the locking member is in engagement with the adjustable member a number of teeth 54 on the locking member 49 are in engagement with corresponding teeth 55 on the main body 41 to lock the adjustable member to the main body in a given position.

The adjustable arm 43 is moveably mounted in a track 56 wherein the adjustable arm 43 may be moved along its longitudinal axis mainly perpendicular to the longitudinal axis of the main body and mainly parallel with the arm 48. The adjustable arm 43 may be locked in a wanted position by means of a strap 57. At one end the strap 57 is preferably hinged to the main body. When the adjustable arm is placed in the wanted position, the strap is forced against the adjustable arm so that not shown teeth or ribs at the strap interact with teeth 58 at the adjustable arm 43. The strap is locked by means of a not shown tongue that is forced into interaction with a not shown notch. It is preferred that the strap is fastened to the main body so that it is not lost before it is used to lock the adjustable arm.

The measuring device 40 is preferably made of a medically approved plastic material allowing the necessary stability and stiffness of the device. The device may then be sterilized and packed in a sterile package. As mentioned above, it is preferred that the locking of both the adjustable member and the adjustable arm is irreversible, meaning that the device is broken and not possible to use again if anybody tries to unlock the device. The measuring device preferably disposable and is thrown after use, to avoid problems in cleaning and sterilization of a used device.

### Surgical procedure

The normal procedure for insertion of an artificial hip joint using the present tool and system is as follows, without being bound of the described sequence of the procedural steps:
1) The patient is examined and a plan for any adjustments of length of the limb, i. e. the length between the floor and hip joint, and offset, i. e. the distance between the longitudinal axis of the femur and the sagittal plane of the body, is made preferably when the patient visits the out patient clicic for preoperative examination.
2) After preparation for surgery the patient is placed on the operating table in a defined "start position", e. g. positioned on the side with the relevant hip up and the legs parallel at the operating table and is supported and stabilised so that the patients trunk and the leg facing down are kept in the same position during the surgical procedure.
3) Stab incisions are made to get access to the femur and pelvis to make bores for fastening the screws3, 3'and the supports 2, 2'. The measuring device 40 is preferably used as a template for making the bores in the femur and the pelvis. The adjustable body 42 is placed at a predetermined distance from the bore 44 taking into account the length required for the prosthesis in question. Then the bores into the femur and pelvis are made using the bores 44 and 45 as drill guide. After making the bores in the pelvis and femur, the screws 3, 3' are entered into the bores using bores 44 and 45 at the measuring device as guides to ensure that the bores in the bones are substantially parallel. Preferably protective sleeves 4,4' are put onto the screw to avoid damage to soft tissue due to contact between the screw and the soft tissue before the supports 2,2' are connected to the screws.
4) The measuring device 40 is then used to measure the distance in three dimensions between the supports 2, 2'. The distance is measured by placing a connecting member 46 at the end of the arm 48, is put into the receptor 5 at the top of the supports 2 and the support is rotated, if necessary, to align the receptor relative to the other support 2'. The adjustable member 42 and the adjustable arm 43 are then adjusted to allow a connection member 47 at the adjustable arm 43 to be put into the receptor 5' at the other support 2'. If necessary the other support 2'is rotated to align the receptor 5'. After placing both the connection members 46, 47 into the receptor 5,5'and assuring that the connection members both are in full contact with the receptor, the position of the adjustable body at the main body 41 and the position of the adjustable arm relative to the main body 41 are read and registered. Any planned adjustment in the length of the limb or offset is then made by adjusting the position of the adjustable body or the adjustable arm, before the adjustable arm and the adjustable body are locked to the main body.
5) The necessary incisions for performing the total hip replacement are then made
6) The neck of femur is divided and the head of femur is removed. The internal femoral canal is then prepared by rasps to receive the stem 31 of the artificial hip joint and the pelvic cavity is hollowed to receive the cup 32 of the artificial hip joint.
7) The rasp or a prosthesis stem 31 is temporarily inserted into the prepared femoral canal. An anteversion head 30 is mounted on the prosthesis stem as illustrated in figure 1 and as described in WO 01/19296. A provisional prosthesis head 33 and a collar 34 are put on the prosthesis neck as an elongation of prosthesis stem 31. The collar 34 and the preliminary head 33 may alternatively be made in one piece and the size of the head may be so large to act as a spacer thus replacing the cup. When a cup is used, the function of the preliminary head and the collar is to interact with the prosthesis cup in that the head rests in a recess in the cup and the collar rests against the surface connecting the recess and the outer surface of the cup, to define the angle between the cup and the prosthesis stem. The prosthesis head 33 and collar 34 are connected to the handle part 14 of the positioning tool 1 by means of the fork member 15 comprising two guide rods 35 that are inserted into guide holes in the collar and/or the guide head. The fork may also be directly connected to the prosthesis, the rasp or extensions thereof.
8) A prosthesis cup is placed into the prepared recess in the pelvis and the artificial joint is put together. The patient is again placed in the start position as during the measurement under the above item 4. The connecting member 46 at the arm 48 is again put into the receptor 5 at the support 2 and the mutual position of the supports is re-established by adjusting the leg of the patient until the connecting member 47 at the adjustable arm 43 rests in the receptor 6 at the other support 2'. The measuring device is then removed and the flexible arms 7,7' are connected to the handle part 14 as described above. The flexible arms 7,7' and the joints 20,20', 21,21', 10 and 10'are adjusted so that the arms are connected to the handle part at the same time as the connection members 6,6'are resting in the receptor 5,5'. The control wheel 11,11' is then tightened so that the flexible arms and the joints are locked in this position. The arms 7,7' are then removed and the artificial joint is disassembled.
9) The anteversion head is connected to the cup 32 of the prosthesis and the handle part 14 is connected to the anteversion head as described above before the cup is placed in the prepared pelvic cavity together with cement to fasten the cup. The other flexible arm not used under step 9) above, is fastened to the top of the handle part as described above and the position of the cup is adjusted until the connection member 6'of the flexible arm 7'rests in the longitudinal axis 5' of the support 2' fastened to pelvis. After recreating the position of the cup relative to the support as under item 8) above, in this way, the cup is held in this position until the cement is hardened sufficiently to remove the anteversion head.
10) The prosthesis stem 31 is cemented into the prepared femoral canal. The position of the prosthesis stem in the femur is controlled by connecting the anteversion head 30 to the prosthesis stem, connecting the handle part to the anteversion head (or to the prosthesis or extensions to the prosthesis) as described above, connecting the arm 7,7' that was used to measure the distance between the top of the handle part and the support on femur, to the top of the handle part and adjusting the position of the prosthesis stem 31 until the connecting member 6 is resting in the receptor. After recreating the position of the prosthesis stem relative to the support as under step 8) above in this way, the position is held until the cement is hardened sufficiently to remove the anteversion head.
11) After replacing the head 33 of the anteversion head with a permanent head for the prosthesis, the leg of the patient is again moved into the basic position to assemble the artificial joint and the surgery is finished in the normal way and the supports and screws are removed.

The prosthesis stem used for provisional insertion in the present description and claims, may be the prosthesis stem that are to be placed permanently into the femur of the patient, it may be a provisional prosthesis stem only used for provisional insertions and measurements or it may be the rasp used for preparing the hollow in the femur for insertion of the prosthesis.

The term start position used in the present description and claims may any position that is useful for performing the surgery and that is easy to control and reproduce. The start position may be with the patient on the side as described above or a position where the patient is lying like a tin soldier having the toes pointing upwards. Additional tools, that are traditionally used to align the patient and control the position, may be used to control and reproduce the start position. The man skilled in the art will recognize which position that is the best suitable start position for a given situation and which tools and techniques to use with regard to surgery and the positioning of the patient.

The surgical procedure described above is the preferred surgical procedure as it allows maximum control even in minimally invasive surgery where the surgeon is performing surgery through minimal incisions and where extra tools are needed to position the prosthesis correctly.

### Alternative surgical procedure I

During this procedure, an acceptable positioning of the parts may be obtained without using the measuring device 40. This procedure comprises the following steps:
1) The patient is examined and a plan for surgery is made, preferably in the out patient clinic.
2) After preparation for surgery the patient is placed at the operating table in a defined "start position", e. g. lying on the side the hip to be operated superior and the legs parallel at the operating table supported and stabilised so that the patients trunk and the leg facing down are kept in the same position during the surgical procedure.
3) Stab incisions are made to get access to the femur and pelvis to make drill canals for fastening the screws 3,3' and the supports 2, 2'. The drill canals into pelvis and femur are made. Preferably a template is used both for the drill when making the drill canals and when fastening the screws to ensure that the drill canals in the bones and subsequently the screws are substantially parallel. Preferably protective sleeves 4,4' are put onto the screw to avoid damage to soft tissue due to contact between the screw and the soft tissue before the supports 2, 2' are connected to the screws.
4) The incisions required for total hip replacement, are then made.
5) The neck of femur is divided and the head of femur is removed. The femoral canal is prepared to receive the stem 31 of the artificial hip joint and the pelvic cavity is prepared to receive the cup 32 of the artificial hip joint.
6) A prosthesis stem 31 is temporarily inserted into the femoral canal. An anteversion head 30 is mounted on the prosthesis stem as illustrated in figure 1 and as described in WO 01/19296. A provisional prosthesis head 33 and a collar 34 are put on the prosthesis neck as an elongation of prosthesis stem 31. The collar 34 and the preliminary head 33 may alternatively be made in one piece. The function of the preliminary head and the collar are to interact with the prosthesis cup in that the head rests in a recess in the cup and the collar rests against the surface connecting the recess and the outer surface of the cup, to define the angle between the cup and the prosthesis stem. In an alternative design, the preliminary head has an outer diameter equal to the cup, thus replacing the cup as a spacer. The prosthesis head 33 and collar 34 are connected to the handle part 14 of the positioning tool 1 by means of the fork member 15 comprising two guide rods 35 that are inserted into guide holes in the collar and/or the guide head.
7) A prosthesis cup is placed into the prepared recess in the pelvis and the artificial joint is put together. The patient is again placed in the start position and the surgeon ensures by visual inspection that the parts of the prosthesis are in place in the hollow in femur and pelvis, respectively. Thereafter it is ensured by means of the anteversion head that the mutual angle between the parts of the prosthesis is correct. The flexible arms 7,7' are then connected to the handle part 14 as described above. The flexible arms 7,7' and the joints 20,20', 21,21', 10 and 10' are adjusted so that the arms are connected to the handle part at the same time as the connection members 6,6'are resting in the receptors 5,5'. The control wheel 11,11' is then tightened so that the flexible arms and the joints are locked in this position. The arms 7,7' are then removed and the artificial joint is disassembled.
8) The anteversion head is mounted on an insertion tool (not shown) and connected to the cup 32 of the prosthesis and the handle part 14 is connected to the anteversion head as described above before the cup is placed in the prepared pelvic cavity together with cement to fasten the cup or without cement for force fit according to the choice of prosthesis. The flexible arm'7, 7' is fastened to the top of the handle part as described above and the position of the cup is adjusted until the connection member 6'of the flexible arm 7'rests in the receptor 5'of the support 2' fastened to pelvis. After recreating the position of the cup relative to the support as under item 8) above, in this way, the cup is held in this position until durable fixation as been obtained e. g. by the cement having hardened sufficiently for the anteversion head to be removed.
9) The prosthesis stem 31 is cemented into the hollowed femur. The position of the prosthesis stem in the femur is controlled by connecting the anteversion head 30 to the prosthesis stem, connecting the handle part to the anteversion head as described above, connecting the other flexible arm 7,7' not used under step 8) above that was used to measure the distance between the top of the handle part and the support at femur, to the top of the handle part and adjusting the position of the prosthesis stem 31 until the connecting member 6 is resting in the receptor. After recreating the position of the prosthesis stem relative to the support as under step 8) above in this way, the position is held until the cement is hardened sufficiently to remove the anteversion head.
10) After replacing the head 33 of the anteversion head with a permanent head for the prosthesis, the leg of the patient is again moved into the start position to assemble the artificial joint and the surgery is finished in the normal way and the supports and screws are removed.

### Alternative surgical procedure II

During this procedure, an acceptable positioning of the parts may be obtained without using the positioning tool 1. This procedure comprises the following steps:
1) The patient is examined and a plan for any adjustments of length of the limb, i.e. the length between the knee and hip joint, and offset, i.e. the distance between the longitudinal axis of the femur and the sagittal plane of the body, is made preferably in the out patient clinic.
2) After preparation for surgery the patient is placed at the operating table in a defined "start position", e. g. lying on the side with the hip to be operated up and the legs parallel at the operating table and supported and stabilised so that the patients trunk and the leg facing down are kept in the same position during the surgical procedure.
3) Stab incisions are made to get access to the femur and pelvis to make drill canals for fastening the screws 3,3' and the supports 2,2'. The measuring device 40 is preferably used as a template for making the drill canals in the femur and the pelvis. The adjustable body 42 is placed at a predetermined distance from the drill guide canal 44. Then the drill canals into the femur and pelvis are made using the bores 44 and 45 as drill guides. After making the bores in the pelvis and femur, the screws 3,3' are entered into the bores using bores 44 and 45 at the measuring device as guides to ensure that the screws in the bones are substantially parallel. Preferably protective sleeves 4,4'are put onto the screw to avoid damage to soft tissue due to contact between the screw and the soft tissue before the supports 2,2'are connected to the screws.
4) The measuring device 40 is then used to measure the distance in two dimensions between the supports 2, 2'. In addition to the distance in two dimensions, the alignment of the supports and ensures correct position also in the third dimension. The distance is measured by placing a connecting member 46 at the end of the arm 48, into the receptor 5 at the top of the supports 2 and the support is rotated, if necessary, to align the receptor relative to the other support 2'. The adjustable member 42 and the adjustable arm 43 are then adjusted to allow a connection member 47 at the adjustable arm 43 to be put into the receptor 5'at the other support 2'. If necessary the other support 2'is rotated to align the receptor5'. After placing both the connection members 46,47 into the receptors 5,5' and assuring that the connection members both are in full contact with the receptors, the position of the adjustable body at the main body 41 and the position of the adjustable arm relative to the main body 41 are read and registered. Any planned adjustment in the length of the limb or offset is then made by adjusting the position of the adjustable body or the adjustable arm, before the adjustable arm and the adjustable body are locked to the main body.
5) The surgical incisions for the total hip replacement, are then made.
6) The neck of femur is divided and the head of femur is removed. The femur is then hollowed to receive the stem 31 of the artificial hip joint and the pelvic cavity is hollowed to receive the cup 32 of the artificial hip joint.
7) Cement is put into the pelvic recess and a prosthesis cup is placed into the recess in the pelvis and the artificial joint is put together. The patient is again placed in the basic position as during the measurement under the above item 4. The connecting member 46 at the arm 48 is again put into the receptor 5 at the support 2 and the mutual position of the supports is re-established by adjusting the leg of the patient until the connecting member 47 at the adjustable arm 43 rests in the receptor 6 at the other support 2'. The hip joint is then held in this position until the cement is hardened.
8) A prosthesis stem 31 is cemented (or force fitted) into the hollow femur. After curing of the cement, an anteversion head 30 is mounted on the prosthesis stem as illustrated in figure 1 and as described in WO 01/19296. A provisional prosthesis head 33 and a collar 34 are put on the prosthesis neck as an elongation of prosthesis stem 31. The collar 34 and the preliminary head 33 may alternatively be made in one piece. The function of the preliminary head and the collar are to interact with the prosthesis cup in that the head rests in a recess in the cup and the collar rests against the surface connecting the recess and the outer surface of the cup, to define the angle between the cup and the prosthesis stem. The prosthesis head 33 and collar 34 are connected to the handle part 14 that substitutes the handle in the in the device according to WO 01/19296 wherein the fork member 15 comprising two guide rods 35 that inserted into guide holes in the collar and/or the guide head.
9) After replacing the head 33 of the anteversion head with a permanent head for the prosthesis, the leg of the patient is again moved into the start position to assemble the artificial joint and the surgery is finished in the normal way and the supports and screws are removed.

Above, the invention is described with reference to the presently preferred embodiments of the system and tool relating to implantation of an artificial hip joint.

The present measuring devices may, however, also be used during other surgical procedures, such as implantation of artificial hinged joints or ball joints, such as an artificial knee prosthesis and other joints.

During control measurements, the prosthesis cup may be substituted by a spacer filling out a space in acetebulum corresponding to the space occupied by the cup.

The prosthesis stem described above has prosthesis neck fixed to the stem. Alternatively a prosthesis stem having a prosthesis neck that is adjustably fixed to the stem, may be used. In using this type of prosthesis stem. The corrections described above for adjusting the position of the prosthesis stem before fixation, may be performed by adjusting the prosthesis neck after fixation of the stem.

## Claims

1. Measuring device for measuring the distance between two supports (5, 5') for use during surgical procedures, where said supports are connected to bones in the patient's body, wherein the measuring device comprises an elongated main body (41);
a first arm (43) connected substantially perpendicular to the longitudinal axis of and close to one end of said main body;
an adjustable member (42) displaceably connected to said main body (41), a second arm (48) being substantially parallel to said first arm (43), connected to the adjustable body (42); and a
connection member (46, 47) arranged at one of the ends of said first (48) and second arm (43), wherein said connection member is adapted to interact with a receptor (5,5') at the supports (2,2'), **characterised in that** said connection member (46, 47) is provided at each of the first (48) and second arm (43).

2. The measuring device according to claim 1, wherein said first arm (43) is displaceably connected to the main body (41) in a direction substantially perpendicular to the longitudinal axis of said main body (41).

3. The measuring device according to claim 1, wherein said second arm (48) is displaceably connected to the adjustable member (42) in a direction substantially perpendicular to the longitudinal axis of said main body (41).

4. Measuring device according to any of the claims 1-3, wherein the adjustable member comprises means to lock the adjustable member to a wanted position along the main body.

5. Measuring device according to claim 4, wherein the adjustable member is adapted to receive and interact with a locking member to lock the adjustable member to the main body.

6. Measuring device according to claim 2, additionally comprising means to lock said first arm in a wanted position.

7. Measuring device according to claim 3, additionally comprising means to lock said second arm in a wanted position.

8. Measuring device according to claim 6 or 7, wherein said means is a strap.

9. The measuring device of any of the preceding claims, wherein substantially parallel bores are made in the main body and the adjustable body.

10. A system for ensuring correct insertion and spatial orientation of a prosthesis cup and/ or a prosthesis stem of an artificial hip joint, the system comprising : a) a tool (30) for controlling the mutual positioning of the main components in a hip prosthesis;
the measuring device (40) of any of claims 1 to 9; and
a detachable positioning tool (1) designed to be connected to a handle part (14) connected to the tool (30) according to item a) or to extensions of the prosthesis components, and to the two supports (5,5') connected to the patients' pelvis and leg.

## Patentansprüche

1. Messvorrichtung zum Messen der Distanz zwischen zwei Stützelementen (5, 5') zur Verwendung während einer Operation, wobei die Stützelemente mit den Knochen im Körper des Patienten verbunden sind, wobei die Messvorrichtung aufweist:
einen länglichen Hauptkörper (41);
einen ersten Arm (43), der mit der Längsachse des Hauptkörpers im Wesentlichen senkrecht und nahe eines Endes dessen mit diesem verbunden ist;
ein verstellbares Element (42), das mit dem Hauptkörper (41) verschiebbar verbunden ist, wobei ein zweiter Arm (48) im Wesentlichen parallel zum ersten Arm (43) verläuft und mit dem verstellbaren Element (42) verbunden ist; und
ein Verbindungsglied (46, 47), das an einem der Enden des ersten Arms (48) und des zweiten Arms (43) angeordnet ist, wobei das Verbindungsglied eingerichtet ist, um mit einem Aufnahmemittel (5, 5') an den Stützelementen (2, 2') zusammenzuwirken, **dadurch gekennzeichnet, dass** das Verbindungsglied (46, 47) sowohl am ersten Arm (48) als auch am zweiten Arm (43) vorgesehen ist.

2. Messvorrichtung nach Anspruch 1, wobei der erste Arm (43) mit dem Hauptkörper (41) in eine Richtung im Wesentlichen senkrecht zur Längsachse des Hauptkörpers (41) verschiebbar verbunden ist.

3. Messvorrichtung nach Anspruch 1, wobei der zweite Arm (48) mit dem verstellbaren Element (42) in eine Richtung im Wesentlichen senkrecht zur Längsachse des Hauptkörpers (41) verschiebbar verbunden ist.

4. Messvorrichtung nach einem der Ansprüche 1 bis 3, wobei das verstellbare Element Mittel zum Verriegeln des selbigen in einer gewünschten Position entlang des Hauptkörpers aufweist.

5. Messvorrichtung nach Anspruch 4, wobei das verstellbare Element eingerichtet ist, um ein Verriegelungselement aufzunehmen und mit diesem zusammenzuwirken, um das verstellbare Element am Hauptkörper zu verriegeln.

6. Messvorrichtung nach Anspruch 2, weiters mit Mitteln zum Verriegeln des ersten Arms in einer gewünschten Position.

7. Messvorrichtung nach Anspruch 3, weiters mit Mitteln zum Verriegeln des zweiten Arms in einer gewünschten Position.

8. Messvorrichtung nach Anspruch 6 oder 7, wobei das Mittel ein Riemen ist.

9. Messvorrichtung nach-einem der vorherigen Ansprüche, wobei der Hauptkörper und das verstellbare Element mit im Wesentlichen parallelen Löchern versehen sind.

10. System zum Sicherstellen, dass eine Prothesenschale und/oder ein Prothesenschaft eines künstlichen Hüftgelenks korrekt eingesetzt und platzmäßig korrekt ausgerichtet wird, wobei das System umfasst:
a) ein Werkzeug (30) zum Steuern des beiderseitigen Positionierens der Hauptkomponenten in einer Hüftprothese;
eine Messvorrichtung (40) nach einem der Ansprüche 1 bis 9; und
ein lösbares Positionierwerkzeug (1), das ausgebildet ist, um mit einem Handgriff (14) verbunden zu werden, wobei der Handgriff (14) mit dem Werkzeug (30) gemäß Punkt a) verbunden ist, oder um mit Verlängerungen der Prothesenkomponenten und mit den beiden Stützelementen (5, 5') verbunden zu werden, die mit dem Becken und dem Bein des Patienten verbunden sind.

## Revendications

1. Dispositif de mesure destiné à mesurer la distance entre deux supports (5, 5'), à utiliser pendant des procédures chirurgicales, où lesdits supports sont reliés aux os dans le corps d'un patient, dans lequel le dispositif de mesure comprend un corps principal allongé (41) ;
un premier bras (43) relié de manière sensiblement perpendiculaire à l'axe longitudinal et proche d'une extrémité dudit corps principal ;
un élément ajustable (42) relié audit corps principal (41) de manière à pouvoir se déplacer, un second bras (48) étant sensiblement parallèle audit premier bras (43), relié au corps réglable (42) ; et
un élément de connexion (46, 47) disposé à l'une des extrémités dudit premier (48) et second (43) bras, dans lequel ledit élément de connexion est adapté pour interagir avec un récepteur (5, 5') au niveau des supports (2, 2'), **caractérisé en ce que** ledit élément de connexion (46, 47) est fourni sur chacun des premier (48) et second (43) bras.

2. Dispositif de mesure selon la revendication 1, dans lequel ledit premier bras (43) est relié, de manière à pouvoir être déplacé, au corps principal (41) dans une direction sensiblement perpendiculaire à l'axe longitudinal dudit corps principal (41).

3. Dispositif de mesure selon la revendication 1, dans lequel ledit second bras (48) est relié, de manière à pouvoir être déplacé, à l'élément ajustable (42), dans une direction sensiblement perpendiculaire à l'axe longitudinal dudit corps principal (41).

4. Dispositif de mesure selon l'une quelconque des revendications 1 à 3, dans lequel l'élément ajustable comprend des moyens pour bloquer l'élément ajustable dans une position souhaitée le long du corps principal.

5. Dispositif de mesure selon la revendication 4, dans lequel l'élément ajustable est adapté pour recevoir et interagir avec un élément de blocage, afin de bloquer l'élément ajustable au corps principal.

6. Dispositif de mesure selon la revendication 2, comprenant en outre des moyens pour bloquer ledit premier bras dans une position souhaitée.

7. Dispositif de mesure selon la revendication 3, comprenant en outre des moyens pour bloquer ledit second bras dans une position souhaitée.

8. Dispositif de mesure selon la revendication 6 ou 7, dans lequel ledit moyen est une bande.

9. Dispositif de mesure selon l'une quelconque des revendications précédentes, dans lequel des alésages sensiblement parallèles sont réalisés dans le corps principal et le corps ajustable.

10. Système destiné à assurer l'insertion correcte et l'orientation dans l'espace d'une tasse de prothèse et/ou d'une tige de prothèse d'une articulation artificielle de hanche, le système comprenant : a) un outil (30) pour contrôler le positionnement réciproque des principaux composants dans une prothèse de hanche ;
le dispositif de mesure (40) selon l'une quelconque des revendications 1 à 9 ; et
un outil de positionnement détachable (1) conçu pour être connecté à une partie de poignée (14) reliée à l'outil (30) selon le point a) ou à des extensions des composants de prothèse, et aux deux supports (5, 5') reliés au bassin et à la jambe des patients.
